# EUROPEAN PATENT APPLICATION

(11) **EP 4 155 263 A1**
(43) Date of publication of application: **29.03.2023**
(21) Application number: 21809700.4
(22) Date of filing: 18.05.2021
(51) Int. Cl.: C01B 32/05, B01J 23/745, B01J 23/89, B01J 35/02

(54) **REACTION SYSTEM, METHOD FOR COLLECTING SOLID CARBON, METHOD FOR PRODUCING GAS CONTAINING HYDROGEN, CATALYST SET, AND CATALYST FOR SOLID CARBON COLLECTION**

(30) Priority: 19.05.2020 JP 2020087256
(71) Applicant: National University Corporation Shizuoka University, Shizuoka-shi, Shizuoka 422-8529 (JP)
(72) Inventor: FUKUHARA, Choji, Hamamatsu-shi, Shizuoka 432-8561 (JP); WATANABE, Ryo, Hamamatsu-shi, Shizuoka 432-8561 (JP)
(74) Representative: Hoffmann Eitle
(86) International application number: PCT/JP2021/018823
(87) International publication number: WO 2021/235443

(57) **Abstract**

Disclosed is a reaction system including: a reforming device including a reaction tube, and a reforming catalyst that is provided inside the reaction tube and generates carbon monoxide from a raw material gas containing hydrocarbon; a solid carbon capturing device including a reaction tube, and a solid carbon capturing catalyst provided inside the reaction tube; and a flow path through which a gas flows from the reforming device to the solid carbon capturing device. The solid carbon capturing catalyst includes a base material, and a coating layer formed on a surface of the base material. The coating layer contains at least one kind of metal-containing component selected from iron oxide, cobalt oxide, and the like.

## Description

### Technical Field

The present disclosure relates to a reaction system, a method for capturing solid carbon, a method for producing gas containing hydrogen, a catalyst set, and a solid carbon capturing catalyst.

### Background Art

To reduce carbon dioxide emissions, a movement to recover and store carbon dioxide is gaining momentum worldwide. From a viewpoint of efficient storage, it is preferable to store carbon dioxide in a state of a solid having a volume smaller in comparison to a gas. Here, development of a technology including methanation of carbon dioxide as in the following reaction formula and precipitation of solid carbon from methane has been in progress (for example, Patent Literature 1, Non-Patent Literature 1).

CO₂ + 4H₂ → CH₄ + 2H₂O (methanation)

CH₄ → C + 2H₂ (solid carbonization)

### Citation List

### Patent Literature

Patent Literature 1: Japanese Unexamined Patent Publication No. 2015-196619

### Non Patent Literature

Non Patent Literature 1: Upham et al., Science, 358, 917-921 (2017) Nov. 2017

### Summary of Invention

### Technical Problem

However, heating at an extremely high temperature of 1000°C or higher is required for a reaction of directly producing solid carbon from hydrocarbon such as methane in many cases. From a viewpoint of reducing carbon dioxide emissions, it is preferable to generate solid carbon at a relatively low temperature.

An aspect of the present disclosure relates to a reaction system capable of efficiently capturing solid carbon from a gas including a carbon source at a relatively low temperature.

### Solution to Problem

An aspect of the present disclosure relates to a solid carbon capturing catalyst comprising: a base material; and a coating layer formed on a surface of the base material. The coating layer contains at least one kind of metal-containing component selected from the group consisting of iron oxide, cobalt oxide, magnesium oxide, molybdenum oxide, nickel oxide, manganese oxide, metallic iron, metallic cobalt, metallic magnesium, metallic molybdenum, metallic nickel, and metallic manganese. For example, the solid carbon capturing catalyst is used in combination with a reforming catalyst that generates carbon monoxide from a raw material gas containing hydrocarbon.

Anther aspect of the present disclosure relates to a solid carbon capturing device including: a reaction tube; and the solid carbon capturing catalyst provided inside the reaction tube.

Still another aspect of the present disclosure relates to a method for capturing solid carbon. The method includes: feeding a raw material gas containing at least one of hydrocarbon and carbon monoxide to the solid carbon capturing device while heating the solid carbon capturing catalyst, thereby causing solid carbon to precipitate onto the solid carbon capturing catalyst.

Sill another aspect of the present disclosure relates to a reaction system including: a reforming device including a reaction tube, and a reforming catalyst that is provided inside the reaction tube and generates carbon monoxide from a raw material gas containing hydrocarbon; and the solid carbon capturing device. A flow path through which a gas flows from the reforming device to the solid carbon capturing device is formed. The reforming catalyst may be a dry reforming catalyst that generates carbon monoxide and hydrogen from a raw material gas containing hydrocarbon and carbon dioxide or may be a steam reforming catalyst that generates carbon monoxide, carbon dioxide, and hydrogen from a raw material gas containing hydrocarbon and water.

Still another aspect of the present disclosure relates to a method for capturing solid carbon. The method includes: feeding a raw material gas containing hydrocarbon to the reaction system while heating the reforming catalyst and the solid carbon capturing catalyst, thereby causing solid carbon to precipitate onto the solid carbon capturing catalyst.

Still another aspect of the present disclosure relates to a method for producing a gas containing hydrogen. The method includes: capturing solid carbon by the method described above; and discharging a discharge gas containing hydrogen from the solid carbon capturing device.

Sill another aspect of the present disclosure relates to a catalyst set including: a reforming catalyst that generates carbon monoxide from a raw material gas containing hydrocarbon; and a solid carbon capturing catalyst. The solid carbon capturing catalyst includes a base material, and a coating layer formed on a surface of the base material. The coating layer contains at least one kind of metal-containing component selected from the group consisting of iron oxide, cobalt oxide, magnesium oxide, molybdenum oxide, nickel oxide, manganese oxide, metallic iron, metallic cobalt, metallic magnesium, metallic molybdenum, metallic nickel, and metallic manganese.

### Advantageous Effects of Invention

According to the aspects of the present disclosure, there are provided a catalyst enabling solid carbon to be efficiently captured at a relatively low temperature, and a solid carbon capturing device and a reaction system which include the catalyst. A combination of the solid carbon capturing device and a reforming device including a reforming catalyst that generates carbon monoxide from a raw material gas containing hydrocarbon realizes efficient capturing of solid carbon, and efficient production of a gas containing hydrogen by a solid carbonization reaction including a Boudoard reaction that generates solid carbon from carbon monoxide.

### Brief Description of Drawings

FIG. 1 is a cross-sectional view illustrating an example of a solid carbon capturing catalyst.
FIG. 2 is a schematic view illustrating an example of a solid carbon capturing device.
FIG. 3 is a schematic view illustrating an example of a reaction system.
FIG. 4 is a schematic view illustrating an example of the reaction system.
FIG. 5 is a graph illustrating a relationship between a dry reforming conversion rate of CH₄ and CO₂ and H₂/CO (molar ratio), and time in a case where a carbon capturing catalyst contains iron oxide (Fe₃O₄).
FIG. 6 is a graph illustrating a carbon capturing rate with respect to a total carbon amount of CH₄ and CO₂ in a raw material gas.
FIG. 7 is a graph illustrating a relationship between a dry reforming conversion rate of CH₄ and CO₂ and H₂/CO (molar ratio), and time in a case where the carbon capturing catalyst contains iron oxide (Fe₃O₄) and a temperature thereof is 450°C, and a relationship between a ratio of hydrogen, carbon dioxide, carbon monoxide, and methane in a discharge gas, and time.
FIG. 8 is a scanning electron micrograph of solid carbon that precipitates onto a carbon capturing catalyst containing Fe₃O₄.
FIG. 9 is a graph illustrating a relationship between a dry reforming conversion rate of CH₄ and CO₂ and H₂/CO (molar ratio), and time in a case where the carbon capturing catalyst contains iron oxide (Fe₂O₃) and a temperature thereof is 470°C.
FIG. 10 is a graph illustrating a relationship between a dry reforming conversion rate of CH₄ and CO₂ and H₂/CO (molar ratio), and time in a case where the carbon capturing catalyst contains Fe/Al₂O₃.
FIG. 11 is a graph illustrating a relationship between a ratio of hydrogen, carbon dioxide, carbon monoxide, and methane in a drying reforming discharge gas and time in a case where the carbon capturing catalyst contains Fe/Al₂O₃.
FIG. 12 is a scanning electron micrograph of solid carbon that precipitates onto the carbon capturing catalyst containing Fe/Al₂O₃.
FIG. 13 is a graph illustrating a relationship between a stream reforming (SRM) conversion rate of CH₄ and H₂/CO (molar ratio), and time in a case where the carbon capturing catalyst contains iron oxide (Fe₃O₄), and a relationship between the amount of hydrogen, carbon dioxide, methane, and carbon monoxide in a discharge gas, and time.

### Description of Embodiments

The present disclosure is not limited by the following examples.

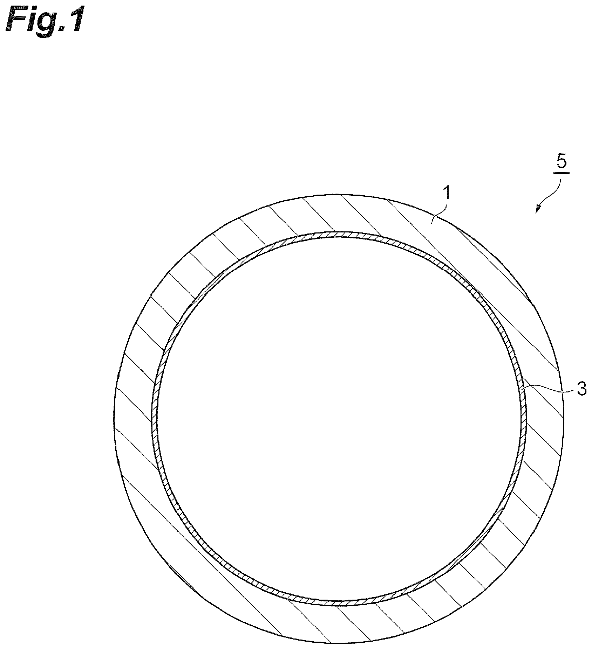

FIG. 1 is a cross-sectional view illustrating an example of a solid carbon capturing catalyst. A solid carbon capturing catalyst 5 illustrated in FIG. 1 includes a base material 1 that is a tubular body, and a coating layer 3 that is formed on an inner wall surface of the base material 1.

In a case where the base material 1 is a tubular body as in an embodiment in FIG. 1, a raw material gas can be easily caused to flow through the inside of the solid carbon capturing catalyst 5. In addition, as a structure that hinders flowing is less on an inner side of the tubular body, clogging due to precipitated solid carbon is less likely to occur. The base material 1 is not particularly limited as long as that base material 1 can be coated with a metal-containing component, but may be, for example, a stainless steel tube or an aluminum tube. When the base material 1 is a tubular body having an inner wall surface with a circular cross-section, an inner diameter thereof is not particularly limited, but the inner diameter may be, for example, 10 to 300 mm or 10 to 1000 mm. When the base material 1 is a tubular body, a length thereof is not particularly limited, but may be, for example, 20 to 5000 mm. The base material 1 may be a tubular body that linearly extends, or may be a twisted tubular body.

A shape of the base material that constitutes the solid carbon capturing catalyst is not limited to the tubular body, and may be an arbitrary shape. For example, the base material may be a planar body (plate-shaped body) or a spiral body, or may be a twisted planar body or a twisted spiral body.

The coating layer 3 contains at least one kind of metal-containing component selected from the group consisting of iron oxide, cobalt oxide, magnesium oxide, molybdenum oxide, nickel oxide, manganese oxide, metallic iron, metallic cobalt, metallic magnesium, metallic molybdenum, metallic nickel, and metallic manganese as a main component. The coating layer 3 may contain iron oxide, particularly, Fe₃O₄, Fe₂O₃, FeO, or a combination hereof as the iron oxide. A ratio of the metal-containing components in the coating layer 3 may be 40 to 100 mass%, 50 to 100 mass%, 60 to 100 mass%, 70 to 100 mass%, 80 to 100 mass%, or 90 to 100 mass% on the basis of the mass of the coating layer 3. The coating layer 3 may contain a carrier that includes aluminum oxide (Al₂O₃) and metallic iron that is carried on the carrier. In this case, a ratio of the metallic iron may be 40 to 50 mass% on the basis of the mass of the coating layer 3.

The coating layer 3 is formed to cover the entirety or a part of a surface (inner wall surface) of the base material 1. The thickness of the coating layer 3 is not particularly limited, but may be, for example, 5 to 2000 µm or 10 to 2000 µm.

The coating layer 3 may be porous from the viewpoint of efficient precipitation of solid carbon. A specific surface area of the porous coating layer may be 5 to 1000 m²/g.

For example, the coating layer may be formed by a method including a step of causing a coating solution containing at least one kind of metal-containing component selected from the group consisting of iron oxide, cobalt oxide, magnesium oxide, molybdenum oxide, nickel oxide, manganese oxide, metallic iron, metallic cobalt, metallic magnesium, metallic molybdenum, metallic nickel, and metallic manganese, or a precursor thereof, and a solvent, to adhere to the surface (for example, the inner wall surface) of the base material 1, and a step of removing the solvent from the coating solution adhered to the surface of the base material 1. The solvent of the coating solution may be, for example, water, alcohol, acetone, or a combination thereof.

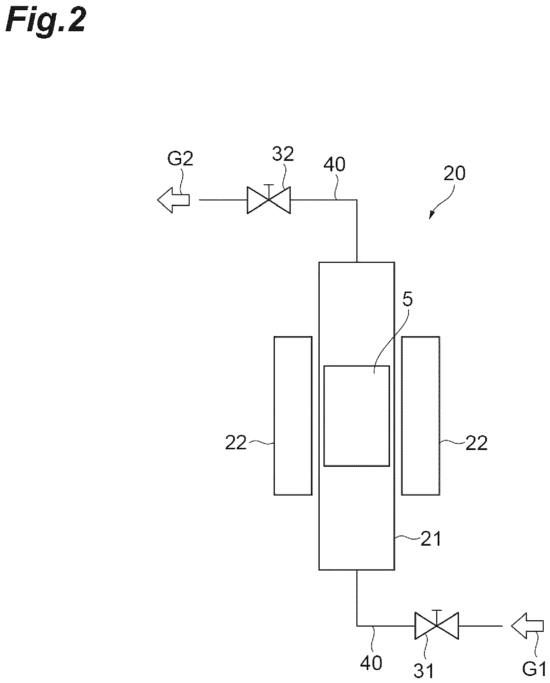

FIG. 2 is a schematic view illustrating an embodiment of a solid carbon capturing device provided with the solid carbon capturing catalyst. A solid carbon capturing device 20 illustrated in FIG. 2 includes a reaction tube 21, and the solid carbon capturing catalyst 5 that is provided inside the reaction tube 21. The solid carbon capturing device 20 further includes a heater 22 that is provided at the periphery of the reaction tube 21. A pipe 40 for gas circulation is connected to each of both ends of the reaction tube 21. A raw material gas G1 is fed from the pipe 40 on one side, and discharge gas G2 is discharged from the pipe 40 on the other side. A valve 31 is provided on the pipe 40 on an upstream side, and a value 32 is provided on the pipe 40 on a downstream side.

When feeding the raw material gas G1 while heating the solid carbon capturing catalyst 5 by the heater 22, solid carbon precipitates onto the coating layer of the solid carbon capturing catalyst 5. The precipitated solid carbon can be easily peeled from the coating layer. For example, a plurality of reaction tubes are prepared, and a reaction tube after capturing carbon and a reaction tube before capturing carbon can be replaced with each other by a revolver method. According to this, solid carbon can be efficiently captured.

The raw material gas G1 includes a carbon compound that generates solid carbon. For example, the raw material gas G1 may include at least either hydrocarbon or carbon monoxide. An element composition of the raw material gas can be adjusted in correspondence with a heating temperature of the solid carbon capturing catalyst 5 or the like in consideration of a relationship between a decomposition temperature of hydrocarbon, and a molar ratio of a carbon atom, a hydrogen atom, and an oxygen atom. Specifically, for example, in a case of 400°C, an O/C ratio may be 0.2 to 2.0 and an H/C ratio may be 0 to 4.3, in a case of 500°C, the O/C ratio may be 0.2 to 2.2 and the H/C ratio may be 0 to 4.7, in a case of 600°C, the O/C ratio may be 0.3 to 2.1 and the H/C ratio may be 0 to 4.8, and in a case of 700°C, the O/C ratio may be 0.3 to 1.9 and the H/C ratio may be 0 to 5.3. Hydrocarbon may include methane. In a case of hydrocarbon having two or more carbon atoms, the hydrocarbon may be used after being decomposed into methane.

A temperature at which the solid carbon capturing catalyst 5 is heated for precipitation of solid carbon may be, for example, 400°C or higher and 800°C or lower, 700°C or lower, 650°C or lower, 550°C or lower, 500°C or lower, or 490°C or lower, or may be 430°C or higher and 800°C or lower, 700°C or lower, 650°C or lower, 550°C or lower, 500°C or lower, or 490°C or lower, or may be 450°C or higher and 800°C or lower, 700°C or lower, 650°C or lower, 550°C or lower, 500°C or lower, or 490°C or lower. The heating temperature may vary constantly during a reaction.

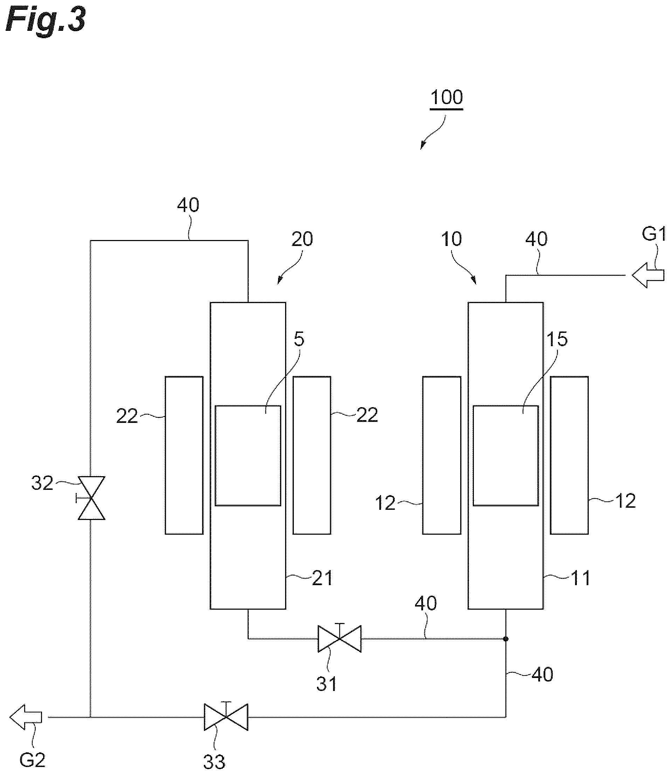

The solid carbon capturing device may be combined with another reaction device that discharges a raw material gas containing a carbon source such as hydrocarbon. FIG. 3 is a schematic view illustrating an example of a reaction system including a combination of the solid carbon capturing device, and a reforming device that includes a reforming catalyst that generates carbon monoxide from the raw material gas containing hydrocarbon. A reaction system 100 illustrated in FIG. 3 mainly includes a reforming device 10, and the solid carbon capturing device 20 according to the above-described embodiment. That is, the reaction system 100 includes a catalyst set constituted by a combination of the reforming catalyst and the solid carbon capturing catalyst. A flow path through which a gas flows from the reforming device 10 to the solid carbon capturing device 20 is formed by a pipe 40. On a downstream side of the reforming device 10, the pipe 40 is branched so that a flow path through which a gas is discharged without passing through the solid carbon capturing device 20 is formed in addition to a flow path toward the solid carbon capturing device 20. A valve 33 is formed on the branched pipe 40.

The reforming device 10 includes a reaction tube 11, a reforming catalyst 15 provided inside the reaction tube 11, and a heater 12 provided at the periphery of the reaction tube 11. For example, the reforming catalyst may be a dry reforming catalyst that generates carbon monoxide and hydrogen from hydrocarbon and carbon dioxide or may be a steam reforming catalyst that generates carbon monoxide, carbon dioxide, and hydrogen from hydrocarbon and water. The reforming catalyst 15 can be heated by the heater 12. The reforming catalyst 15 includes a base material that is a tubular body, and a reforming catalyst activation component (for example, a dry reforming catalyst activation component or a steam reforming catalyst activation component) disposed on the base material. The base material may include a honeycomb-structure fin accommodated on an inner side of the tubular body, and a catalyst activation layer containing the reforming catalyst activation component may be formed on a surface of the fin. For example, the catalyst activation layer may contain a porous carrier containing alumina, and a reforming catalyst activation component carried on the porous carrier. For example, the reforming catalyst activation component may be nickel, cobalt, molybdenum, rhodium, ruthenium, aluminum, zirconium, magnesium, or oxides thereof, or nickel, cobalt, molybdenum, rhodium, aluminum, or oxides thereof. The reforming catalyst activation components can function as a dry reforming reaction catalyst or a steam reforming reaction catalyst to be described later. Other examples of the reforming catalyst activation component for the steam reforming reaction include palladium, zinc, potassium, and calcium. The reforming catalyst 15 may be a granular body containing the reforming catalyst activation component.

In a case where the reforming catalyst 15 is the dry reforming catalyst, when a raw material gas G1 containing hydrocarbon and carbon dioxide is fed to the reaction system 100 while heating the reforming catalyst 15 (or the reforming catalyst activation component) and the solid carbon capturing catalyst 5, carbon monoxide and hydrogen are generated by the following dry reforming reaction in the reforming catalyst 15. In a case where the reforming catalyst 15 is the steam reforming catalyst, when a raw material gas G1 containing hydrocarbon and water (steam) is fed to the reaction system 100 while heating the reforming catalyst 15 (or the reforming catalyst activation component) and the solid carbon capturing catalyst 5, carbon monoxide and hydrogen are generated by the following steam reforming reaction in the reforming catalyst 15. The reforming catalyst 15 may be subjected to a reducing treatment by a hydrogen gas or the like before feeding the raw material gas G1.

CH₄ + CO₂ → 2CO + 2H₂ (drying reforming reaction)

Methane + water → carbon dioxide + carbon monoxide + hydrogen (steam reforming reaction)

The raw material gas discharged from the reforming device 10 contains carbon monoxide and hydrogen in addition to hydrocarbon. When the raw material gas flows into the solid carbon capturing device 20, a Boudoard reaction of generating solid carbon from carbon monoxide also progresses in addition to a decomposition reaction of hydrocarbon (for example, methane), and thus it is considered that solid carbon efficiently precipitates.

CH₄ → C + 2H₂ (solid carbonization reaction, decomposition reaction of methane)

2CO → C + CO₂ (solid carbonization reaction, Boudoard reaction)

In a case where solid carbon is captured mainly by the Boudoard reaction, a ratio of water in a discharge gas G2 is small.

A heating temperature of the reforming catalyst 15 (or the reforming catalyst activation component) may be, for example, 500°C to 900°C.

The reaction system 100 can be used to produce a gas containing hydrogen simultaneously with capturing of solid carbon. Particularly, when the reforming catalyst 15 is the steam reforming catalyst, a gas (discharge gas G2) containing hydrogen at a high ratio can be efficiently produced. Recently, hydrogen has attracted attention as next generation energy, and a use amount thereof tends to increase. In the related art, 15 billion m³ of hydrogen has been supplied by using the steam reforming, but 22 billion m³ of hydrogen will be required to be supplied in the future. A supply amount by water electrolysis known as an additional hydrogen supply method is limited to approximately 2000 m³/h in the current performance. With regard to steam reforming, it is preferable to further increase a ratio of hydrogen and to reduce carbon dioxide emissions. An increase in a ratio of hydrogen and a decrease in a ratio of carbon dioxide in a discharge gas can be realized due to a combination of the reforming device including the steam reforming catalyst and the solid carbon capturing device.

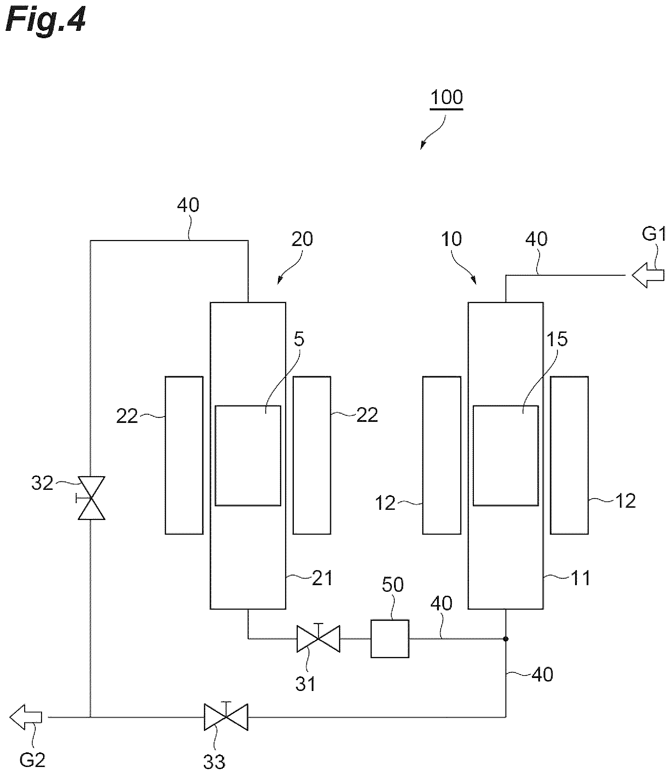

In a case where the reforming catalyst 15 is the steam reforming catalyst, as in an additional example illustrated in FIG. 4, the reaction system 100 may include a moisture removing device 50 provided on a flow path (pipe 40) that connects the reforming device 10 and the solid carbon capturing device 20 to each other. When the moisture removing device 50 is provided, the amount of moisture in a gas that flows into the solid carbon capturing device 20 decreases, and according to this, an addition improvement can be realized from the viewpoints of an increase in the amount of solid carbon captured, a decrease in carbon dioxide emissions, and an increase in a ratio of a hydrogen gas in the discharge gas G2.

The raw material gas G1 that is fed to the reforming device 10 may be a gas that contains methane generated by the following methanization reaction of CO₂. Accordingly, the reaction system 100 may further include a methanization reaction device provided on an upstream side of the reforming device 10.

CO₂ + 4H₂ → CH₄ + 2H₂O (methanization reaction)

### Examples

Hereinafter, the invention will be described in more detail with reference to examples. However, the invention is not limited to the examples.

### - Examination 1 -

### 1. Preparation of Reaction System

### 1-1. Dry Reforming Catalyst

80 mL of water in a beaker was heated to 55°C, and 12 g of aluminum triisopropoxide was added to the water. The reaction solution in the beaker was stirred for 5 minutes, and was left as is for 20 minutes, and then 6 mL of nitric acid and 4 mL of formaldehyde were sequentially added to the reaction solution. The reaction solution was stirred for several minutes, and was left as is. Next, the reaction solution was stirred for several minutes every 20 minutes, the reaction solution was cooled down to room temperature at a point of time after passage of 100 minutes after addition of formaldehyde. Leaving the reaction solution as is at room temperature for 2 days resulted in forming an aluminum sol.

A stainless steel tube (formed from ferrite-based stainless steel, diameter: 20 mm, length: 60 mm) in which a honeycomb-structure fin (formed from ferrite-based stainless steel) is accommodated was used as a base material for forming a catalyst. The base material was immersed sequentially in an aqueous sodium hydroxide solution and an aqueous hydrochloric acid solution for activation. The activated base material was immersed in the aluminum sol. The base material taken out from the aluminum sol was rotated on a rotation stage to make the aluminum sol adhered to the base material have a uniform thickness. Next, the base material to which the aluminum sol adhered was baked by a muffle furnace to form an alumina porous body on a surface of the base material. The same operation was repeated three times.

The base material on which the alumina porous body was formed was immersed alternately in an Sn bath and a Pd bath to form Pd cores in the alumina porous body. Next, Ni was caused to precipitate onto the Pd cores by electrolysis plating to obtain the dry reforming catalyst including the alumina porous body and a catalyst activation layer containing metallic nickel carried on the alumina porous body.

### 1-2. Solid Carbon Capturing Catalyst (Fe₃O₄)

Iron oxide (Fe₃O₄) was finely pulverized through grinding and was suspended in propanol. A hollow stainless steel tube (formed from ferrite-based stainless steel, diameter: 20 mm, length: 20 to 50 mm) was immersed in the obtained suspension. The stainless steel tube was taken out from the suspension, the suspension adhered to the stainless steel tube was dried by heating at 60°C to 100°C to form a porous coating layer (thickness: 10 to 2000 µm) containing Fe₃O₄ on an inner wall surface of the stainless steel tube. One to five stainless steel tubes on which a similar coating layer is formed were prepared as the solid carbon capturing catalyst.

### 2-1. Catalyst Reaction Test (1)

### Example 1

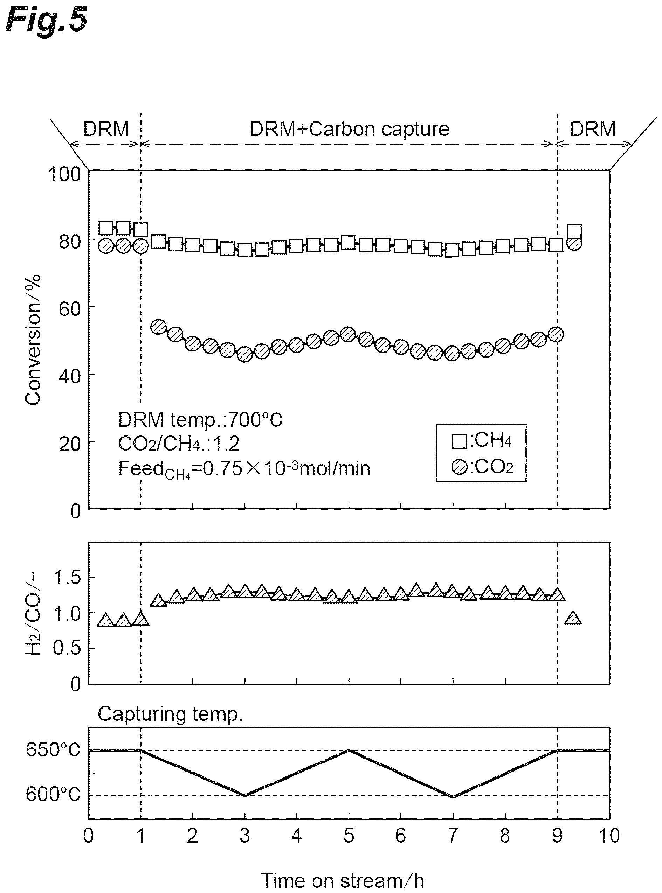

The prepared dry reforming catalyst and the prepared solid carbon capturing catalyst were provided in a reaction tube of a reaction system including a reforming device and a solid carbon capturing device having a similar configuration as in FIG. 3. One to five carbon capturing catalysts were arranged in series within the reaction tube. A CO₂ cylinder, a CH₄ cylinder, an N₂ cylinder, and an H₂ cylinder were connected to a pipe through which the raw material gas is fed. The valves 31 and 32 were opened, and the valve 33 was closed for a hydrogen reducing treatment of the catalyst, and the temperature of the dry reforming catalyst and the solid carbon capturing catalyst was maintained at 600°C for 2 hours while flowing a hydrogen gas. Next, the valve 31 and the valve 32 on a pipe on the solid carbon capturing device side were closed, and the valve 33 was opened, the raw material gas containing CH₄ and CO₂ was caused to flow to the reaction tube of the dry reforming device for 1 hour while heating the dry reforming catalyst at 700°C thereby performing only a dry reforming reaction of methane (DRM). A composition of the raw material gas was adjusted so that a flow rate of CH₄ becomes 0.75×10⁻³ moles/minute and CO₂/CH₄ (molar ratio) becomes 1.2. Next, the valve 31 and the valve 32 on the pipe on the solid carbon capturing device side were opened and the valve 33 was closed, and the raw material gas was caused to flow to the dry reforming reaction tube and the carbon capturing reaction tube for 8 hours. During this process, lowering of the temperature of the carbon capturing catalyst from 650°C to 600°C for 2 hours, and raising of the temperature from 600°C to 650°C for 2 hours were repeated two times. Then, the valve on the pipe on the solid carbon capturing catalyst was closed, and the raw material gas was caused to flow to only the dry reforming reaction tube for 1 hour. In the above-described processes, a composition of a discharge gas was analyzed by TCD gas chromatograph (GC-8A, manufactured by SHIMADZU CORPORATION). FIG. 5 is a graph illustrating a relationship between a conversion rate of CH₄ and CO₂ and H₂/CO (molar ratio), and time. It could be confirmed that a large amount of precipitated solid carbon adhered to the inside of the carbon capturing catalyst taken out from the reaction tube. A carbon capturing rate was 15.5% with respect to a total carbon amount of CH₄ and CO₂ in the raw material gas.

### Comparative Example 1

A catalyst reaction test was performed in a similar manner as in the example except that one to five stainless steel tubes (formed from ferrite-based stainless steel, diameter: 20 mm, length: 20 to 50 mm) that does not include a coating layer were used as is instead of the catalyst including the coating layer that contains iron oxide. The carbon capturing rate was 8.5% with respect to the total carbon amount of CH₄ and CO₂ in the raw material gas.

### 2-2. Catalyst Reaction Test (2)

### Example 2

The carbon capturing rate was measured under the same conditions as in Example 1 except that the temperature of the carbon capturing catalyst was maintained at 450°C, 460°C, 470°C, 480°C, 490°C, 500°C, 550°C, 600°C, or 650°C for 8 hours while the raw material gas was caused to flow to the dry reforming reaction tube and the carbon capturing reaction tube.

### Comparative Example 2

A catalyst reaction test was performed in a similar manner as in Example 2 except that stainless steel tubes (formed from ferrite-based stainless steel, diameter: 20 mm, length: 20 to 50 mm) that does not include a coating layer were used as is instead of the catalyst including the coating layer that contains iron oxide, and the temperature was maintained at 600°C for 8 hours.

### Result

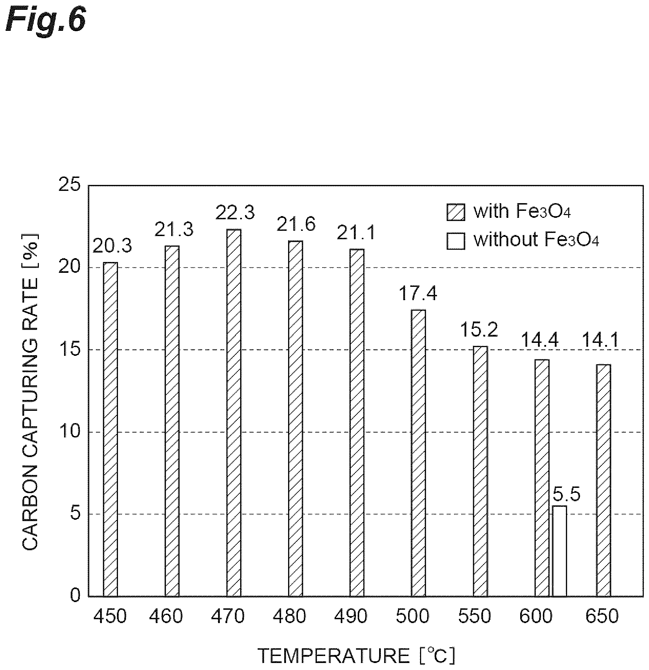

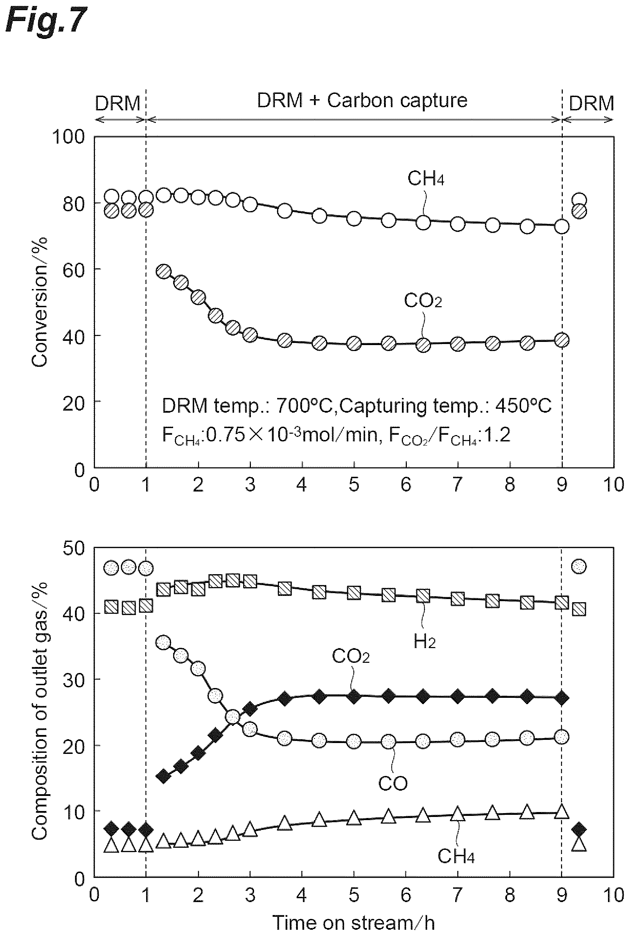

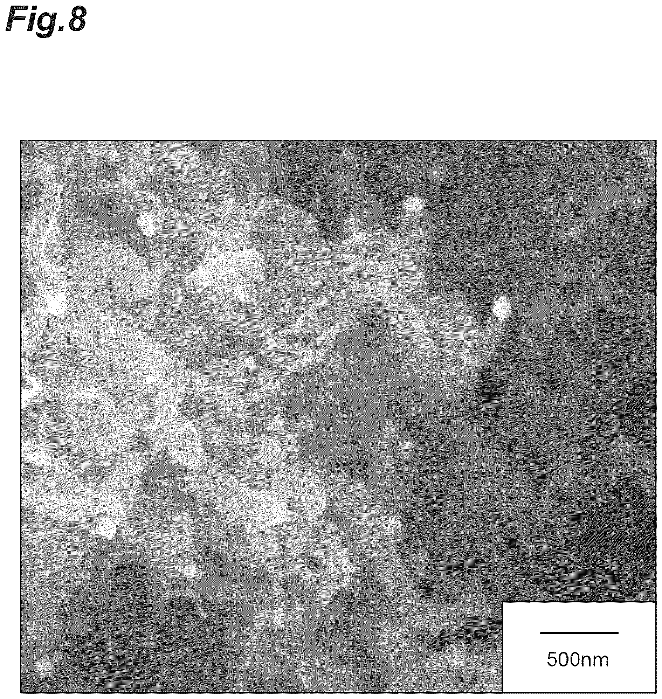

FIG. 6 is a graph illustrating the carbon capturing rate with respect to the total carbon amount of CH₄ and CO₂ in the raw material gas. FIG. 7 is a graph illustrating a relationship between a conversion rate of CH₄ and CO₂ and H₂/CO (molar ratio), and time in a case where the temperature of the carbon capturing catalyst is 450°C, and a relationship between a ratio of hydrogen, carbon dioxide, carbon monoxide, and methane in the discharge gas, and time. FIG. 8 is a scanning electron micrograph of solid carbon that precipitates onto the carbon capturing catalyst containing Fe₃O₄.

### - Examination 2 -

### 1. Solid Carbon Capturing Catalyst

### Fe₂O₃, FeO, and Cobalt Oxide

A solid carbon capturing catalyst including a stainless steel tube and a porous coating layer containing metal oxides on an inner wall surface of the stainless steel tube was prepared in a similar method as in "1-2. Solid Carbon Capturing Catalyst (Fe₃O₄)" except that iron oxide (Fe₂O₃), iron oxide (FeO), or cobalt oxide (a mixture of CoO and Co₃O₄) was used instead of iron oxide (Fe₃O₄).

### Fe/Al₃O₄

3 g of high-purity alumina and 30 mL of distilled water were put into a side-arm flask, a mixed solution inside the flask was stirred for 5 hours while being degassed to prepare an alumina slurry. Iron nitrate nonahydrate (20.7 mg) and 30 mL of distilled water were put into a 200 mL beaker and the resultant mixture was stirred for 10 minutes to obtain an aqueous iron nitrate solution. The alumina slurry was added to the aqueous iron nitrate solution and the mixed solution inside the beaker was stirred for 2 hours. Then, the mixed solution was transferred to an evaporation dish, was heated to 80°C, and a moisture was evaporated while stirring the mixed solution with a fluorine resin rod. A powder remained on the evaporation dish was baked by heating at 600°C for 3 hours while flowing air by using a circulation type firing device to obtain a catalyst (Fe/Al₂O₃) in which metallic iron was carried on the alumina carrier.

Fe/Al₂O₃ (6 g) and 2-propanol (40 mL) were put into a polypropylene bottle to prepare a slurry containing Fe/Al₂O₃. A stainless steel hollow tube (outer diameter: 2.0 cm, inner diameter: 1.8 cm, and length: 2.5 cm) whose mass was measured in advance was immersed in the obtained slurry for 5 seconds. The hollow tube taken out from the slurry was dried by a drier, a portion adhered to an outer wall in Fe/Al₂O₃ adhered to a surface of the hollow tube was wiped off with a paper wipe to obtain a solid carbon capturing catalyst including a coating layer containing Fe/Al₂O₃ on an inner wall surface of the hollow tube. The weight of the obtained solid carbon capturing catalyst was weighed again, and the mass of the hollow tube before immersion in the slurry was subtracted from the value of the weight to calculate the net mass of Fe/Al₂O₃ (the mass of the coating layer). Immersion into the slurry and drying were repeated until the mass of Fe/Al₂O₃ reached 50 mg.

### 2. Catalyst Reaction Test

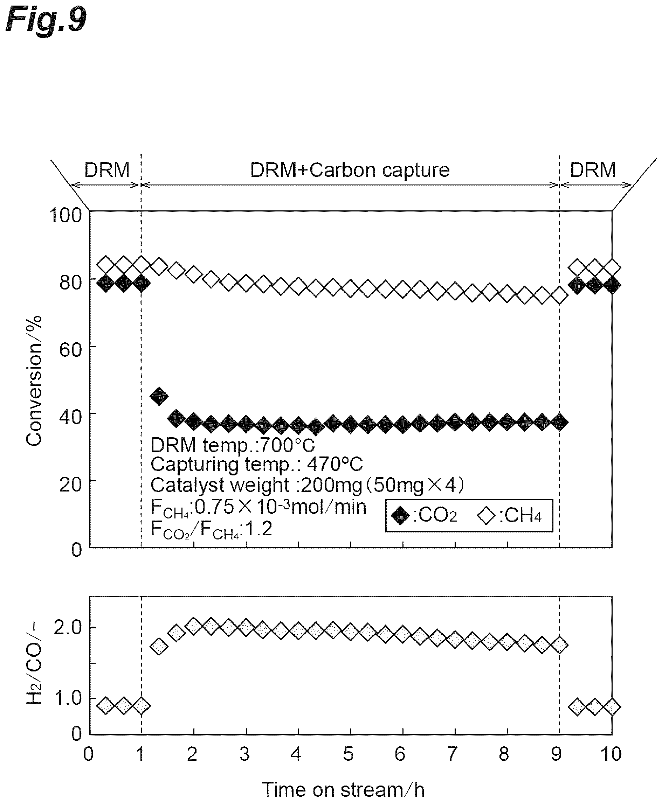

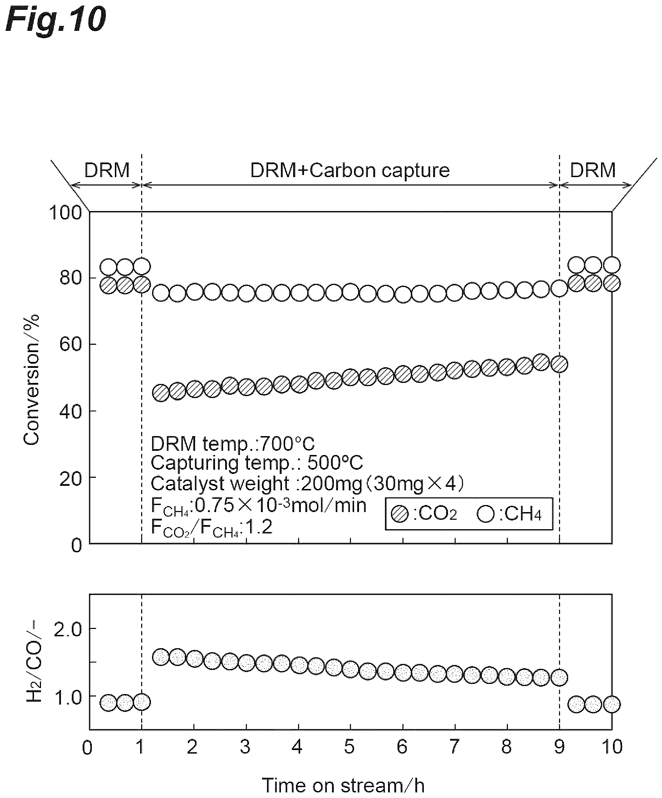

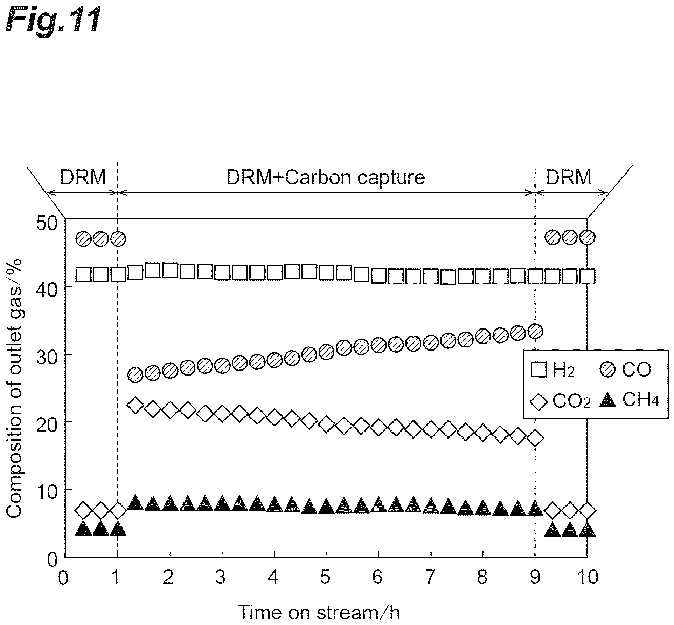

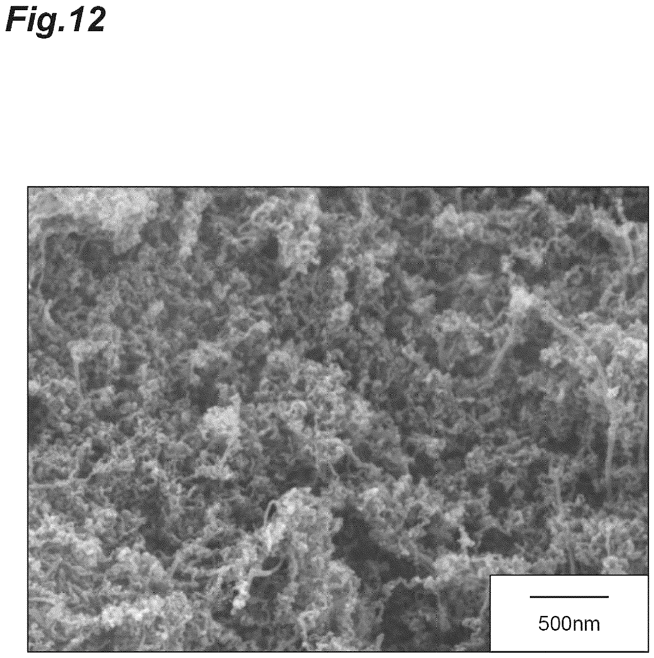

The carbon capturing rate was measured under similar conditions as in Example 1 except that the prepared solid carbon capturing catalyst was used, and the temperature of the carbon capturing catalyst was maintained at a predetermined temperature for 8 hours while the raw material gas was caused to flow to the dry reforming reaction tube and the carbon capturing reaction tube. In a case of the iron oxide (Fe₂O₃), the temperature of the carbon capturing catalyst was maintained at 450°C, and the carbon capturing rate was 20.3% with respect to the total carbon amount of CH₄ and CO₂ in the raw material gas. In a case of the iron oxide (FeO), the temperature of the carbon capturing catalyst was maintained at 600°C, and the carbon capturing rate was 15.0% with respect to the total carbon amount of CH₄ and CO₂ in the raw material gas. In a case of the cobalt oxide, the temperature of the carbon capturing catalyst was maintained at 500°C, and the carbon capturing rate was 10.2% with respect to the total carbon amount of CH₄ and CO₂ in the raw material gas. A composition of a discharge gas in a case where the carbon capturing catalyst contains iron (III) oxide (Fe₂O₃), and a case where the carbon capturing catalyst contains Fe/Al₂O₃ was analyzed by TCD gas chromatograph (GC-8A, manufactured by SHIMADZU CORPORATION). In a case of Fe/Al₂O₃, the temperature of the carbon capturing catalyst was maintained at 500°C, and the carbon capturing rate was 16.0% with respect to the total carbon amount of CH₄ and CO₂ in the raw material gas. FIG. 9 is a graph illustrating a relationship between the conversion rate of CH₄ and CO₂ and H₂/CO (molar ratio), and time in a case where the carbon capturing catalyst contains iron oxide (Fe₂O₃). FIG. 10 is a graph illustrating a relationship between the conversion rate of CH₄ and CO₂ and H₂/CO (molar ratio), and time in a case where the carbon capturing catalyst contains Fe/Al₂O₃. FIG. 11 is a graph illustrating a relationship between a ratio of hydrogen, carbon dioxide, carbon monoxide, and methane in a discharge gas and time in a case where the carbon capturing catalyst contains Fe/Al₂O₃. FIG. 12 is a scanning electron micrograph of solid carbon that precipitates onto the carbon capturing catalyst containing Fe/Al₂O₃.

### - Examination 3 -

### Catalyst Reaction Test (Steam Reforming)

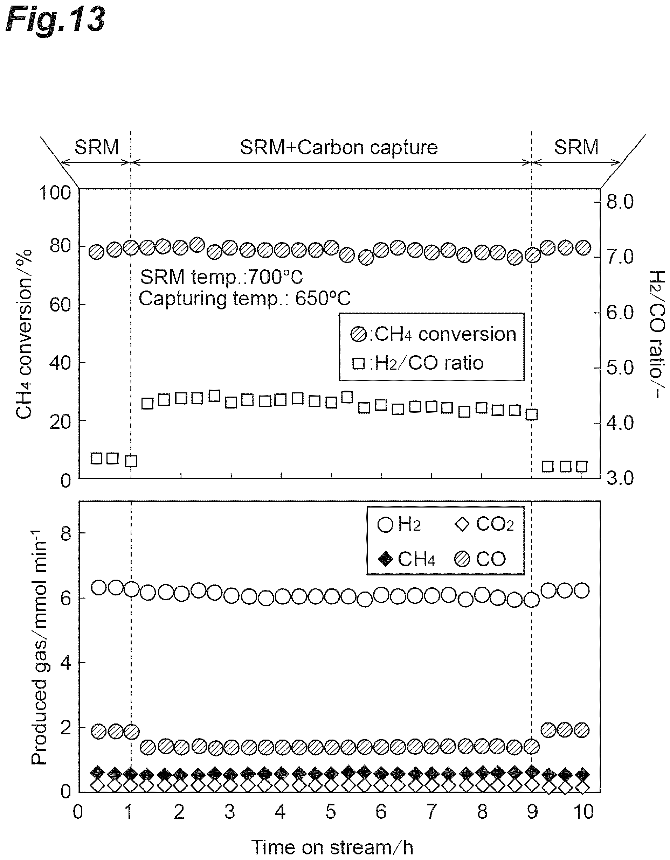

In the same reaction system as in Example 1, an HPLC pump that feeds water, a CH₄ cylinder, an N₂ cylinder, and an H₂ cylinder were connected to a pipe through which the raw material gas is fed were connected to the pipe through which the raw material gas is fed. The valves 31 and 32 were opened, and the valve 33 was closed for a hydrogen reducing treatment of the catalyst, and the temperature of the reforming catalyst and the solid carbon capturing catalyst was maintained at 600°C for 2 hours while flowing a hydrogen gas. Next, the valve 31 and the valve 32 on a pipe on the solid carbon capturing device side were closed, and the valve 33 was opened, the raw material gas containing CH₄ and CO₂ was caused to flow to the reaction tube of the reforming device for 1 hour while heating the reforming catalyst at 700°C thereby performing only a steam reforming reaction of methane (SRM). A composition of the raw material gas was adjusted so that a flow rate of CH₄ and H₂O becomes 2.5×10⁻³ moles/minute and CO₂/CH₄ (molar ratio) becomes 1.0. Next, the valve 31 and the valve 32 on the pipe on the solid carbon capturing device side were opened and the valve 33 was closed, and the raw material gas was caused to flow to the reforming reaction tube and the carbon capturing reaction tube for 8 hours. During this process, the temperature of the carbon capturing catalyst was maintained at 650°C. Then, the valve on the pipe on the solid carbon capturing catalyst was closed, and the raw material gas was caused to flow to only the reforming reaction tube for 1 hour. In the above-described processes, a composition of a discharge gas was analyzed by TCD gas chromatograph (GC-8A, manufactured by SHIMADZU CORPORATION). FIG. 13 is a graph illustrating a relationship between a conversion rate of CH₄ and CO₂, and H₂/CO (molar ratio), and time, and a relationship between the amount of hydrogen, carbon dioxide, methane, and carbon monoxide in a discharge gas, and time. The carbon capturing rate was 18.0% with respect to a total carbon amount of CH₄ and CO₂ in the raw material gas. A discharge gas containing hydrogen at a high ratio was produced.

### Reference Signs List

1: base material, 3: coating layer, 5: solid carbon capturing catalyst, 10: reforming device, 11: reaction tube, 12: heater, 15: reforming catalyst, 20: solid carbon capturing device, 21: reaction tube, 22: heater, 40: pipe, 50: moisture removing device, 100: reaction system, G1: raw material gas, G2: discharge gas.

## Claims

1. A reaction system, comprising:
a reforming device comprising a reaction tube, and a reforming catalyst that is provided inside the reaction tube and generates carbon monoxide from a raw material gas containing hydrocarbon;
a solid carbon capturing device comprising a reaction tube, and a solid carbon capturing catalyst provided inside the reaction tube; and
a flow path through which a gas flows from the reforming device to the solid carbon capturing device,
wherein the solid carbon capturing catalyst comprises a base material, and a coating layer formed on a surface of the base material, and
the coating layer contains at least one kind of metal-containing component selected from the group consisting of iron oxide, cobalt oxide, magnesium oxide, molybdenum oxide, nickel oxide, manganese oxide, metallic iron, metallic cobalt, metallic magnesium, metallic molybdenum, metallic nickel, and metallic manganese.

2. The reaction system according to claim 1,
wherein the base material is a tubular body, a planar body, or a spiral body.

3. The reaction system according to claim 1,
wherein the base material is a tubular body, and the coating layer is formed on an inner wall surface of the tubular body.

4. The reaction system according to claim 3,
wherein the tubular body is a stainless steel tube.

5. The reaction system according to any one of claims 1 to 4,
wherein the coating layer is porous.

6. The reaction system according to any one of claims 1 to 5,
wherein the coating layer contains Fe₃O₄.

7. The reaction system according to any one of claims 1 to 6,
wherein the reforming catalyst is a dry reforming catalyst that generates carbon monoxide and hydrogen from a raw material gas containing hydrocarbon and carbon dioxide.

8. The reaction system according to any one of claims 1 to 6,
wherein the reforming catalyst is a steam reforming catalyst that generates carbon monoxide, carbon dioxide, and hydrogen from a raw material gas containing hydrocarbon and water.

9. The reaction system according to any one of claims 1 to 8,
wherein the reforming catalyst comprises a base material, and a catalyst activation layer formed on a surface of the base material, and
the catalyst activation layer contains at least either at least one kind of metal selected from the group consisting of nickel, cobalt, molybdenum, rhodium, ruthenium, aluminum, zirconium, and magnesium, or an oxide thereof.

10. The reaction system according to any one of claims 1 to 9,
wherein the reaction system is used to capture solid carbon from a gas containing methane generated by a methanation reaction of CO₂.

11. A method for capturing solid carbon, comprising:
feeding a raw material gas containing hydrocarbon to the reaction system according to any one of claims 1 to 10 while heating the reforming catalyst and the solid carbon capturing catalyst, thereby causing solid carbon to precipitate onto the solid carbon capturing catalyst.

12. The method according to claim 11, further comprising:
generating methane by a methanation reaction of CO₂,
wherein the raw material gas containing the methane is fed to the reaction system.

13. A method for producing a gas containing hydrogen, the method comprising:
capturing solid carbon by the method according to claim 11 or 12; and
discharging a discharge gas containing hydrogen from the solid carbon capturing device.

14. A catalyst set, comprising:
a reforming catalyst that generates carbon monoxide from a raw material gas containing hydrocarbon; and
a solid carbon capturing catalyst,
wherein the solid carbon capturing catalyst comprises a base material, and a coating layer formed on a surface of the base material, and
the coating layer contains at least one kind of metal-containing component selected from the group consisting of iron oxide, cobalt oxide, magnesium oxide, molybdenum oxide, nickel oxide, manganese oxide, metallic iron, metallic cobalt, metallic magnesium, metallic molybdenum, metallic nickel, and metallic manganese.

15. The catalyst set according to claim 14,
wherein the base material is a tubular body, a planar body, or a spiral body.

16. The catalyst set according to claim 14,
wherein the base material is a tubular body, and the coating layer is formed on an inner wall surface of the tubular body.

17. The catalyst set according to claim 16,
wherein the tubular body is a stainless steel tube.

18. The catalyst set according to any one of claims 14 to 17,
wherein the coating layer is porous.

19. The catalyst set according to any one of claims 14 to 18,
wherein the coating layer contains Fe₃O₄.

20. The catalyst set according to any one of claims 14 to 19,
wherein the reforming catalyst is a dry reforming catalyst that generates carbon monoxide and hydrogen from a raw material gas containing hydrocarbon and carbon dioxide.

21. The catalyst set according to any one of claims 14 to 19,
wherein the reforming catalyst is a steam reforming catalyst that generates carbon monoxide, carbon dioxide, and hydrogen from a raw material gas containing hydrocarbon and water.

22. The catalyst set according to any one of claims 14 to 21,
wherein the reforming catalyst includes a base material, and a catalyst activation layer formed on a surface of the base material, and
the catalyst activation layer contains at least either at least one kind of metal selected from the group consisting of nickel, cobalt, molybdenum, rhodium, ruthenium, aluminum, zirconium, and magnesium, or an oxide thereof.

23. A solid carbon capturing catalyst that is used in combination with a reforming catalyst that generates carbon monoxide from a raw material gas containing hydrocarbon, comprising:
a base material; and
a coating layer formed on a surface of the base material,
wherein the coating layer contains at least one kind of metal-containing component selected from the group consisting of iron oxide, cobalt oxide, magnesium oxide, molybdenum oxide, nickel oxide, manganese oxide, metallic iron, metallic cobalt, metallic magnesium, metallic molybdenum, metallic nickel, and metallic manganese.
